(19) Europäisches Patentamt
European Patent Office
Office européen des brevets

(11) Veröffentlichungsnummer: **0 027 555**
**B1**

(12) # EUROPÄISCHE PATENTSCHRIFT

(45) Veröffentlichungstag der Patentschrift: **04.01.84**

(51) Int. Cl.³: **C 07 C 93/14**, C 07 C 103/50, C 07 C 109/04, C 07 C 121/75, C 07 C 149/42 // A01N33/22, A01N37/34, A01N37/44

(21) Anmeldenummer: **80105658.1**

(22) Anmeldetag: **20.09.80**

(54) **Verfahren zur Herstellung von Diphenyläthern.**

(30) Priorität: **24.09.79 DE 2938595**

(43) Veröffentlichungstag der Anmeldung: **29.04.81 Patentblatt 81/17**

(45) Bekanntmachung des Hinweises auf die Patenterteilung: **04.01.84 Patentblatt 84/1**

(84) Benannte Vertragsstaaten: **AT BE CH DE FR GB IT LI LU NL SE**

(56) Entgegenhaltungen:
**EP - A - 0 007 482**
**DE - A - 1 543 326**
**DE - A - 2 418 295**
**DE - A - 2 753 235**
**DE - A - 2 800 105**
**US - A - 3 914 310**
**US - A - 4 039 588**
**US - A - 4 046 798**

**BERICHTE DER DEUTSCHEN CHEMISCHEN GESELLSCHAFT, Band 56, Nr. 6, 6. Juni 1923, Berlin und Leipzig. W. BORSCHE, "Über den Austausch von OR gegen andere Radikale in Nitrophenol-äthern, Seiten 1488 bis 1493**
**Patents Abstracts of Japan, Band 3, Nr. 109, 12. September 1979, Seite 85C58**

(73) Patentinhaber: **CELAMERCK GmbH & Co. KG, Binger Strasse 173, D-6507 Ingelheim am Rhein (DE)**

(72) Erfinder: **Sehring, Richard, Dr., Frankenstrasse 13, D-6507 Ingelheim/Rhein (DE)**
Erfinder: **Buck, Wolfgang, Dr., In der Dörrwies 37, D-6507 Ingelheim/Rhein (DE)**

(56) Entgegenhaltungen: (Fortsetzung)
**Patents Abstracts of Japan, Band 3, Nr. 128, 24. Oktober 1979, Seite 27C62**
**Patents Abstracts of Japan, Band 4, Nr. 101, 19 Juli 1980, Seite 82C19**

**EP 0 027 555 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Diphenyläthern

Die Erfindung betrifft ein neues Verfahren zur Herstellung von Diphenyläthern der allgemeinen Formel

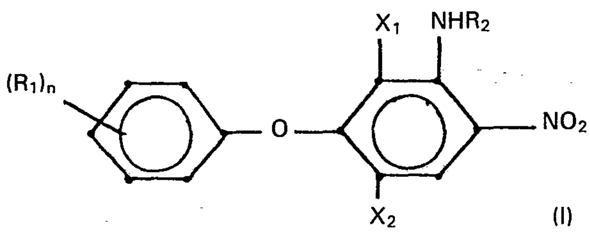

(I)

in der

n eine ganze Zahl von 1 bis 3,

$R_1$ Wasserstoff, Halogen, Alkyl mit 1 bis 8 C-Atomen, Trifluormethyl, Acetyl, CN, $C_1$-$C_4$-Alkoxy, $C_1$-$C_4$-Alkylthio,

$R_2$ Wasserstoff; Alkyl, Alkenyl oder Alkinyl mit bis zu 18 C-Atomen; einen durch Hydroxy, $C_1$-$C_4$-Alkoxy, Phenoxy, Halogenphenoxy, $C_1$-$C_4$-Alkylphenoxy, $C_1$-$C_4$-Alkoxyphenoxy, Nitrophenoxy, Cyanophenoxy, Amino oder $C_1$-$C_4$-Alkylthio substituierten Alkylrest mit 2 bis 6 C-Atomen; gegebenenfalls halogen- oder $CF_3$-substituiertes Benzyl, $NR_3R_4$, $CHR_3$-$COOR_4$ oder $CHR_3$-$CONR_4R_5$ bedeutet, wobei $R_3$, $R_4$ und $R_5$, die gleich oder verschieden sein können, Wasserstoff oder $C_1$-$C_8$-Alkylreste darstellen, und

$X_1$ und $X_2$, die gleich oder verschieden sein können, für Wasserstoff oder Halogen stehen.

Die Werte 2 und 3 nimmt n nur an, wenn $R_1$ wenigstens zum Teil für Halogen oder Alkyl steht.

Unter «Halogen» sind Fluor, Chlor, Brom und Jod zu verstehen. Bevorzugt sind Fluor, Chlor und Brom, vor allem Fluor und Chlor.

Soweit $R_1$, $R_2$, $R_3$, $R_4$, $R_5$ Kohlenwasserstoffreste darstellen oder enthalten, können diese verzweigt oder unverzweigt sein.

Im Rahmen der vorstehenden Definitionen steht $R_1$ bevorzugt für Wasserstoff, Fluor, Chlor, Brom oder Methyl, $R_2$ für Wasserstoff, $C_1$-$C_4$-Alkyl, Allyl, 2-Hydroxyäthyl 4-Fluor- oder 4-Chlorbenzyl oder Dimethylamino. $R_3$ ist vorzugsweise Wasserstoff oder Methyl, $R_4$ $C_1$-$C_4$-Alkyl. Für $X_1/X_2$ sind hauptsächlich die Bedeutungspaare Chlor/Wasserstoff, Chlor/Brom und Brom/Chlor hervorzuheben.

Bedeutet n 2 oder 3, können verschiedene Reste $R_1$ gleichzeitig vorliegen.

Einige Verbindungen der allgemeinen Formel I sind bekannt (EP-A-0 007 482). Sie zeichnen sich durch hervorragende herbizide Wirkung aus. Zur Herstellung dient die Umsetzung von Phenolaten mit halogenierten Nitroanilinen. Die Nitroaniline müssen isomerenfrei und frei von Diaminen sein.

Als Lösungsmittel dienen z.B. Acetonitril, Dimethylformamid, Dimethylsulfoxyd.

N-substituierte Nitroaniline führen leicht zu Nebenreaktionen und damit zu verminderten Ausbeuten.

In «Berichte der deutschen chem. Ges.», Bd. 56, Seiten 1486 - 1493, wird u.a. die Umsetzung von Diphenoxyverbindungen des Typs

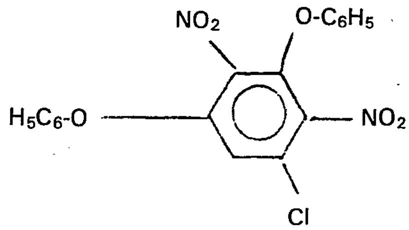

mit Aminen bzw. Hydrazin (S. 1488) beschrieben.

In diesen Verbindungen werden beim Erwärmen mit alkoholischer Hydrazinlösung die verätherten Phenylhydroxyle glatt durch $NHNH_2$ ersetzt. Bei dieser Reaktion dienen jedoch Dinitroverbindungen und nicht Mononitroverbindungen als Ausgangsstoffe, und es wird auch nicht nur eine, sondern es werden beide Phenoxygruppen ersetzt.

Der erfindungsgemässe Reaktionsverlauf war aus dieser Literaturstelle nicht abzuleiten.

Wie nun gefunden wurde, können die Verbindungen der allgemeinen Formel I überraschenderweise in sehr guter Ausbeute und nahezu isomerenrein erhalten werden, indem man ein Nitrodiphenoxybenzol der allgemeinen Formel

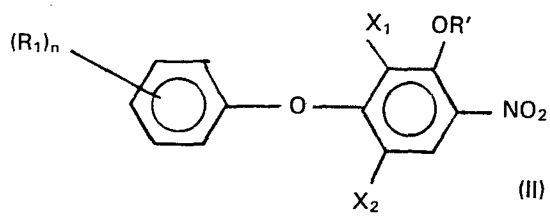

(II)

in der n, $R_1$, $X_1$ und $X_2$ die obige Bedeutung haben und R' für einen gegebenenfalls substituierten Phenylrest steht, mit einem Amin der allgemeinen Formel

$$HNHR_2 \qquad (III)$$

bei Temperaturen zwischen 20°C und 160°C umsetzt.

Der Rest R' steht im allgemeinen für

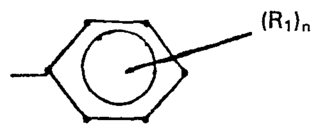

(IV)

jedoch kann er auch einen anders substituierten Phenylrest darstellen, da etwa vorhandene Substituenten, sofern sie unter den Reaktionsbedingungen hinreichend stabil sind, auf den Reaktionsablauf keinen wesentlichen Einfluss haben.

Als Reaktionsmedium können inerte organische Lösungsmittel, beispielsweise Dioxan, Tetrahydrofuran, Benzol, Toluol, Xylol, chlorierte Kohlenwasserstoffe, Dimethylsulfoxid, aber auch Wasser Verwendung finden. Die Auswahl des Reaktionsmediums ist nicht kritisch, jedoch wird bevorzugt ein

Medium benutzt, in dem die Reaktionskomponenten eine ausreichende Löslichkeit besitzen.

Gegenüber dem Verfahren der EP-A-0 007 482 liegt ein Vorteil des neuen Verfahrens darin, dass die Ausgangsstoffe der Formel II in geeigneter Qualität leichter herstellbar sind als die für das ältere Verfahren benötigten Chlornitroaniline und dass auch mit wohlfeilen Lösungsmitteln als Reaktionsmedium gearbeitet werden kann, z.B. mit Wasser, wobei sehr gute Ausbeuten erhalten werden.

Die Ausgangsstoffe der Formel II sind z.T. bekannt. Sie können jedoch auch in neuer Weise hergestellt werden, indem man eine Verbindung der allgemeinen Formel

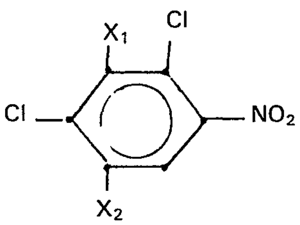

(V)

worin $X_1$ und $X_2$ wie oben definiert sind, z.B. 2,3,4-Trichlornitrobenzol und 2,3,4,5-Tetrachlornitrobenzol, mit einem entsprechenden Phenolat der allgemeinen Formel

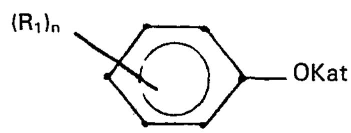

(VI)

(Kat gleich 1 Äquivalent eines Kations) umsetzt. Bevorzugt setzt man die Alkaliphenolate ein, insbesondere das Natriumphenolat, bzw. das Phenol und z.B. ein Alkalicarbonat. Bei einfachen Phenolen, vor allem beim unsubstituierten Phenol, kann das entsprechende Phenol als Reaktionsmedium dienen. Besonders günstig ist jedoch die Verwendung von Dimethylsulfoxyd als Lösungsmittel.

Die beiden gegen Phenoxygruppen austauschbaren Chloratome in 2- und 4-Stellung zeigen deutlich abgestufte Reaktionsfähigkeit. Daher ist es ohne weiteres möglich, Verbindungen mit zwei verschiedenen Phenoxygruppen zu synthetisieren. dazu wird bei milderen Bedingungen zunächst das der Nitrogruppe benachbarte Chloratom gegen den gewünschten Phenoxyrest ausgetauscht, bevor unter schärferen Bedingungen das 4-Chloratom durch die Gruppe der Formel VI ersetzt wird.

Die Verbindungen der allgemeinen Formel I sind teilweise neu. Diese neuen Verbindungen zeigen ebenfalls eine hervorragende herbizide Wirksamkeit und können ausserdem als Zwischenprodukte für die Herstellung neuer Pestizidwirkstoffe oder neuer Arzneistoffe dienen, da ihre funktionellen Gruppen vielfältige Abwandlungen ermöglichen.

Herstellung der Ausgangsstoffe:

1. *2-Chlor-4-nitro-1,3-diphenoxybenzol*

a) 114,5 g 2,3,4-Trichlornitrobenzol, 400 ml Dimethylsulfoxyd und 130 g Natriumphenolat werden 1 Stunde bei 100°C gerührt. Nach dieser Zeit wird das Reaktionsgemisch mit Wasser versetzt und das Chloroform ausgeschüttelt. Man wäscht die Chloroformlösung mit verdünnter Natronlauge, trocknet mit Natriumsulfat und entfernt das Lösungsmittel. Der Rückstand wird aus Äthanol umkristallisiert.

Ausbeute: 152 g (89% d.Th.);
Fp. 106 - 107°C.

b) 56,7 g 2,3,4-Trichlornitrobenzol (0,25 Mol), 94 g Phenol und 34,5 g Kaliumcarbonat (0,25 Mol) werden 1 Stunde auf 130 - 140°C erhitzt. Man gibt das Reaktionsgemisch auf 2 n Natronlauge. Das Reaktionsprodukt wird in einer Ausbeute von über 95% der Theorie erhalten. Fp. 106 - 107°C (aus Äthanol).

2. *6-Chlor-4-nitro-1,3-diphenoxybenzol*

Man erhitzt 1 Mol 2,4,5-Trichlor-1-nitrobenzol mit 2 Mol Kaliumphenolat in Phenol auf 165°C und isoliert das Reaktionsprodukt.

Fp. 95°C (aus Äthanol).

3. *2,6-Dichlor-4-nitro-1,3-diphenoxybenzol*

26,2 g (0,1 Mol) 2,3,4,5-Tetrachlor-1-nitrobenzol werden mit 20,7 g Phenol und 30 g Kaliumcarbonat in 100 ml Dimethylsulfoxyd 4 Stunden auf 110°C erhitzt. Das Reaktionsgemisch wird auf Wasser gegeben und das Produkt abgesaugt. Ausbeute: 33,5 g (89% d.Th.); Fp. 152°C (aus Eisessig).

Dieselbe Verbindung erhält man aus 52,4 g 2,3,4,5-Tetrachlornitrobenzol, 122 g Phenol und 60 g Kaliumcarbonat, indem man die Bestandteile 1 1/2 Stunden auf 160°C erhitzt, die heisse Mischung in 1,2 Ltr. 2 n Natronlauge einrührt und das ausgeschiedene Produkt absaugt. Nach dem Umkristallisieren aus 300 ml Eisessig erhält man 70,5 g der gewünschten Verbindung (93,8% d.Th.); Fp. 164°C.

4. *2-Chlor-4-nitro-5-phenoxy-1-(2,4,5-trichlorphenoxy)-benzol*

28,4 g 4,5-Dichlor-2-nitro-1-phenoxybenzol, 21,6 g 2,4,5-Trichlorphenol, 10 ml 10 n Natronlauge und 80 ml Dimethylsulfoxyd werden 6 Stunden bei 150°C gerührt, dann über Nach bei Raumtemperatur stehen gelassen. Man verrührt das Reaktionsgemisch mit 600 ml Wasser, macht alkalisch und nimmt das Reaktionsprodukt in Chloroform auf. Die organische Phase wird gewaschen, getrocknet und eingeengt. Der Rückstand wird aus 200 ml Äthanol umkristallisiert. Man erhält 43 g hellgelbe Kristalle (97% d.Th.); Fp. 84°C.

5. *2-Chlor-4-nitro-5-phenoxy-1-(2,5-dichlor-4-bromphenoxy)-benzol*

Man gibt 28,4 g 4,5-Dichlor-2-nitro-1-phenoxybenzol, 24,2 g 2,5-Dichlor-4-bromphenol, 10 ml 10 n Natronlauge und 80 ml Dimethylsulfoxyd zusammen und rührt 5 Stunden bei 1000°C. Das Reaktionsgemisch wird dann mit 700 ml 1 n Natronlauge verrührt, über Nacht bei Raumtemperatur stehen gelassen, abdekantiert und das Reaktionsprodukt in Chloroform aufgenommen. Man wäscht die Lösung, trocknet mit Natriumsulfat und engt ein. Der Rückstand wird aus 200 ml Äthanol plus 2 ml Dimethylformamid umkristallisiert. Ausbeute: 36,5 g hellgelbe Kristalle (75% d.Th.); Fp. 85°C.

6. *2-Chlor-4-nitro-3-phenoxy-1-(2,5-dichlor-4--methylthiophenoxy)-benzol*

14,2 g 2,3-Dichlor-6-nitro-1-phenoxybenzol, 10,2 g 2,5-Dichlor-4-methylthiophenol, 5,0 ml 10 n Natronlauge und 40 ml Dimethylsulfoxyd werden 5 Stunden bei 100°C gerührt, über Nacht stehen gelassen und dann mit 400 ml 1 n Natronlauge vermischt. Das abgeschiedene Produkt wird aus 150 ml Äthanol umkristallisiert.

Ausbeute: 20,5 g hellgelbe Kristalle (90% d.Th.); Fp. 138 - 144°C.

7. *2-Chlor-4-nitro-3-phenoxy-1-(2,4,5-trichlorphenoxy)-benzol*

28,4 g 2,3-Dichlor-6-nitro-1-phenoxybenzol, 21,6 g 2,4,5-Trichlorphenol, 10 ml 10 n Natronlauge und 80 ml Dimethylsulfoxyd werden 6 Stunden bei 100°C gerührt und danach mit 600 ml 1 n Natronlauge vermischt. Man saugt ab und kristallisiert das Produkt aus 150 ml Äthanol um.

Ausbeute: 31 g (77,4% d.Th.);
Fp. 112°C.

Einen weiteren Anteil des Reaktionsprodukts (ca. 6 g) kann man durch Einengen der Mutterlauge gewinnen.

8. *2-Chlor-4-nitro-3-phenoxy-1-(4-cyanophenoxy)--benzol*

14,2 g 2,3-Dichlor-6-nitro-1-phenoxybenzol, 6,5 g 4-Cyanophenol, 40 ml Dimethylsulfoxyd und 5 ml 10 n Natronlauge werden 3 Stunden bei 100°C gerührt. Man verdünnt mit Wasser, schüttelt mit Chloroform aus, trocknet und destilliert das Lösungsmittel ab. Nach Reinigung über eine Kieselgelsäule und Umkristallisation aus Äthanol erhält man 11 g farblose Kristalle (60,2% d.Th.);
Fp. 140 - 142°C.

Die folgenden Beispiele erläutern das erfindungsgemässe Verfahren:

*Beispiel 1*

*2-Chlor-3-phenoxy-6-nitro-N-[1-(methylcarbamoyl)-äthyl]-anilin*

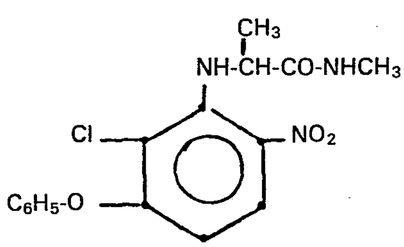

63,3 g 2-Chlor-4-nitro-1,3-diphenoxybenzol werden bei 70°C 24 Stunden in 200 ml Dioxan mit 60 g α-Alanin-N-methylamid gerührt. Das Dioxan wird abdestilliert, der Rückstand in Äthylenchlorid gelöst und die Lösung mit verdünnter Natronlauge ausgeschüttelt. Nach dem Trocknen der organischen Phase mit Natriumsulfat wird das Lösungsmittel abdestilliert. Der Rückstand wird aus Äthanol umkristallisiert.

Ausbeute: 61,5 g (87,2% d.Th.); Fp. 122°C.

Das Produkt ist dünnschichtchromatographisch einheitlich.

Analyse:

| | | | |
|---|---|---|---|
| ber.: | C 54,62 | H 4,55 | Cl 10,10 |
| gef.: | C 54,30 | H 4,41 | Cl 10,07 |

*Beispiel 2*

*2-Chlor-3-phenoxy-6-nitro-N-[2-(2,5-dichlorphenoxy)-äthyl]-anilin*

Man verfährt entsprechend Beispiel 1, verwendet jedoch anstelle des Alanin-methylamids [2-(2,5-dichlorphenoxy)-äthyl]-amin. Auch die Aufarbeitung entspricht Beispiel 1. Das Reaktionsprodukt ist ein Öl, das in einer Ausbeute von 97% d.Th. erhalten wird. Es ist dünnschichtchromatographisch einheitlich.

Analyse:

| | | | |
|---|---|---|---|
| ber.: | C 52,92 | H 3,31 | Cl 23,46 |
| gef.: | C 52,87 | H 3,24 | Cl 23,7 |

*Beispiel 3*

*4-Chlor-3-phenoxy-6-nitro-N-allylanilin*

63,3 g 6-Chlor-4-nitro-1,3-diphenoxybenzol werden mit überschüssigem Allylamin in 120 ml Dioxan 12 Stunden auf 60°C erwärmt. Nach dem Abdestillieren des Lösungsmittels löst man den Rückstand in Methylenchlorid, wäscht mit verdünnter Natronlauge, trocknet die organische Phase mit Natriumsulfat und destilliert das Lösungsmittel ab. Nach dem Umkristallisieren aus Toluol/Hexan, gegebenenfalls weiterer Reinigung über eine Kieselgelsäule, erhält man das Reaktionsprodukt in einer Ausbeute von 51 g (83,6 d.Th.); Fp. 81 - 82°C.

Analyse:

| | | | |
|---|---|---|---|
| ber.: | C 59,11 | H 4,27 | Cl 11,66 |
| gef.: | C 58,9 | H 4,32 | Cl 11,80 |

*Beispiel 4*

*4-Chlor-3-phenoxy-6-nitro-N-(2-aminoäthyl)-anilin--hydrochlorid*

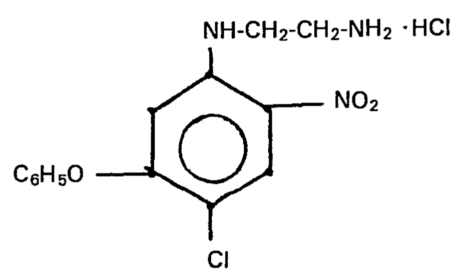

6,83 g 6-Chlor-4-nitro-1,3-diphenoxybenzol werden in 15 ml Dioxan gelöst und mit überschüssigem 1,2-Diaminoäthan 12 Stunden auf 70°C erwärmt. die Aufarbeitung entspricht Beispiel 3.

Zur Reinigung wird aus verdünnter Salzsäure umkristallisiert.

Ausbeute: 6,1 g (88% d.Th.); weisse Kistalle, Fp. 255°C (Zers.).

*Beispiel 5*

*3-Phenoxy-6-nitro-N-allylanilin*

10 g 4-Nitro-1,3-diphenoxybenzol werden in

30 ml Tetrahydrofuran und 10 ml Allylamin 8 Stunden zum Rückfluss erhitzt. die Aufarbeitung erfolgt analog Beispiel 3. Das erhaltene Produkt wird aus Äthanol umkristallisiert.

Ausbeute 6,1 g (69% d.Th.); Fp. 67 - 68°C.

Nach dem Dünnschichtchromatogramm (Cyclohexan/Toluol 1 : 1) liegt ein einheitliches Produkt vor.

Analyse:

| | | | |
|---|---|---|---|
| ber.: | C 66,67 | H 5,19 | Cl 10,37 |
| gef.: | C 66,85 | H 5,30 | Cl 10,24 |

*Beispiel 6*

*2-Chlor-3-phenoxy-6-nitroanilin*

a) 68,3 g 2-Chlor-4-nitro-1,3-diphenoxybenzol werden in 300 ml Toluol gelöst und im Autoklav mit 100 ml konz. wässrigem Ammoniak versetzt. Man stellt dann mit gasförmigem Ammoniak den Druck auf 3 bar. Die Mischung wird sodann 12 Stunden auf 120°C erhitzt, wobei der Druck auf ca. 13 bar ansteigt. Nach dem Abkühlen wird die organische Phase abgetrennt, mit verdünnter Natronlauge ausgeschüttelt, getrocknet und das Lösungsmittel abdestilliert. Das Reaktionsprodukt wird in nahezu quantitativer Ausbeute erhalten (54 g). Nach der gaschromatographischen Untersuchung enthält das Produkt 98% an Titelverbindung. Fp. 81 - 82°C.

b) Der Ansatz entspricht a), jedoch wird kein gasförmiges Ammoniak eingeleitet. Die Reaktionstemperatur beträgt 150°C. Man arbeitet auf wie vorstehend beschrieben. Die Ausbeute ist nahezu quantitativ. Nach der gaschromatographischen Untersuchung besteht das Produkt zu 96% aus der Titelverbindung und zu ca. 4% aus Ausgangsmaterial.

*Beispiel 7*

*2-Chlor-3-(2,5-dichlor-4-methylthiophenoxy)-6-nitro-N-benzylanilin*

Eine Mischung aus 4,6 g 2-Chlor-4-nitro-3-phenoxy-1-(2,5-dichlor-4-methylthiophenoxy)-benzol, 3,0 g Benzylamin und 10 ml Dioxan werden 6 Stunden bei 40°C gerührt, über Nacht bei Raumtemperatur stehengelassen und danach eingeengt. Man nimmt in Chloroform auf, schüttelt die Lösung mit verdünnter Natronlauge und danach Wasser aus, trocknet die abgetrennte organische Phase und engt sie ein. Das erhaltene dunkelbraune Öl wird über eine Kieselgelsäule gereinigt.

Ausbeute 4,2 g eines orangefarbenen viskosen Öls, das dünnschichtchromatographisch einheitlich ist.

*Beispiel 8*

*4-Chlor-3-(2,5-dichlor-4-bromphenoxy)-6-nitro-N-allylanilin*

Man mischt 4,9 g 2-Chlor-4-nitro-5-phenoxy-1-(2,5-dichlor-4-bromphenoxy)-benzol, 1,8 g Allylamin und 10 ml Dioxan. Die Mischung wird zunächst 7 Stunden bei 40°C, dann noch 3 Stunden bei 60°C gerührt und eingeengt. Man nimmt den Rückstand in Chloroform auf, wäscht die Lösung sauer und dann alkalisch, trocknet und engt ein. Das Rohprodukt (5,6 g) wird über eine Kieselgelsäule gereinigt.

Ausbeute: 3,0 g gelbe Kristalle (66,6% d.Th.); Fp. 102,5°C.

*Beispiel 9*

*4-Chlor-3-(2,5-dichlor-4-bromphenoxy)-6-nitro-N-benzylanilin*

Eine Mischung aus 4,9 g 2-Chlor-4-nitro-5-phenoxy-1-(2,5-dichlor-4-bromphenoxy)-benzol, 3,0 g Benzylamin und 10 ml Dioxan wird entsprechend Beispiel 8 umgesetzt. Das Rohprodukt (6,2 g) wird mit 30 ml Äthanol heiss verrührt, dann nach dem Abkühlen auf Raumtemperatur abgesaugt. Man erhält 3,2 g dunkelgelbe Kristalle; Fp. 157°C.

*Beispiel 10*

*2-Chlor-3-(2,5-dichlor-4-methylthiophenoxy)-6-nitro-N-allylanilin*

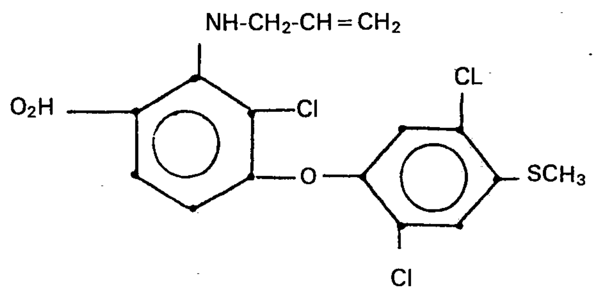

Eine Mischung aus 4,6 g 2-Chlor-4-nitro-3-phenoxy-1-(2,5-dichlor-4-methylthiophenoxy)-benzol, 1,8 g Allylamin und 10 ml Dioxan wird 6 Stunden bei 40°C gerührt, dann über Nacht bei Raumtemperatur stehengelassen. Man engt die Mischung ein, nimmt in Chloroform auf und wäscht sauer und alkalisch. Nach dem Trocknen wird die organische Phase eingeengt. Man erhält 5,2 g Rohprodukt, das aus 20 ml Äthanol umkristallisiert wird.

Ausbeute: 3,8 g orangefarbene Kristalle (91% d. Th.); Fp. 107°C.

*Beispiel 11*

*2-Chlor-3-phenoxy-6-nitro-N-(4-chlorbenzyl)-anilin*

5 g 2-Chlor-4-nitro-1,3-diphenyloxybenzol werden in 10 ml Dioxan mit 6 g 4-Chlorbenzylamin 12 Stunden bei 80°C gerührt. Die Mischung wird dann eingeengt, der Rückstand in Chloroform aufgenommen, die Lösung mit 1 n Natronlauge ausgeschüttelt und über Natriumsulfat getrocknet. Nach dem Abdestillieren des Lösungsmittels wird der Rückstand aus Äthanol umkristallisiert. Ausbeute 4,7 g (82,7% d.Th.), Fp. 95 - 96°C.

Analog erhält man 2-Chlor-3-phenoxy-6-nitro-N-(4-fluorbenzyl)-anilin; gelbe Kristalle, Ausbeute 4,1 g (75,4% d.Th.); Fp. 75 - 76°C.

*Beispiel 12*

*2-Chlor-3-phenoxy-6-nitro-N-(3-trifluormethylbenzyl)-anilin*

3,41 g 2-Chlor-4-nitro-1,3-diphenoxybenzol werden in 5 ml Dioxan mit 4 g 3-Trifluormethylbenzylamin 12 Stunden bei 100°C gerührt. Nach dem

Einengen wird in Chloroform aufgenommen, mit 2 n Natronlauge und 2 n Salzsäure ausgeschüttelt und die organische Phase über Natriumsulfat getrocknet. Beim Abdestillieren des Lösungsmittels erhält man die Titelverbindung als dickflüssiges gelbes Öl (4,0 g; 95% d.Th.).

Analyse:

| | | | |
|---|---|---|---|
| ber.: | C 56,8 | H 3,32 | Cl 6,64 |
| gef.: | C 56,5 | H 3,57 | Cl 6,47 |

*Beispiel 13*

*4-Chlor-3-phenoxy-6-nitro-N-(3-trifluormethylbenzyl)-anilin*

Analog Beispiel 12 erhält man aus 3,41 g 6-Chlor-4-nitro-1,3-diphenoxybenzol und 4 g 3-Trifluormethylbenzylamin in 5 ml Dioxan (20 Stunden bei 100°C) die Titelverbindung in einer Ausbeute von 3 g (71,2% d.Th.), Fp. 140 : 142°C (aus Äthanol).

*Beispiel 14*

*2-Chlor-3-(4-cyanophenoxy)-6-nitro-N-allylanilin*

Eine Mischung aus 4 g 2-Chlor-4-nitro-3-phenoxy-1-(4-cyanophenoxy)-benzol, 10 ml Dioxan und 2 g Allylamin wird 30 Stunden bei Raumtemperatur stehen gelassen. Dann wird das Lösungsmittel abdestilliert, der Rückstand in Chloroform aufgenommen, mit verdünnter Natronlauge ausgeschüttelt, die organische Phase über Natriumsulfat getrocknet, das Chloroform abdestilliert und der Rückstand aus Äthanol umkristallisiert.

Ausbeute 3,5 g (97,2% d.Th.), gelbe Kristalle, Fp. 85 - 87°C.

*Beispiel 15*

*2-Chlor-3-phenoxy-6-nitro-N',N'-dimethylphenylhydrazin*

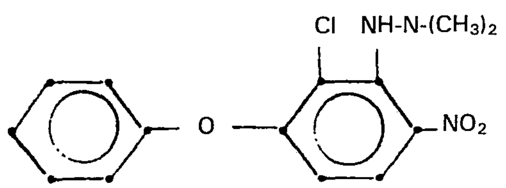

Eine Mischung aus 6,85 g 2-Chlor-4-nitro-1,3-diphenoxy-benzol, 20 ml Dioxan und 9 g N,N-Dimethylhydrazin wird 10 Stunden unter Rückfluss gekocht. Man destilliert das Lösungsmittel ab, nimmt in Chloroform auf, wäscht mit verdünnter Natronlauge und trocknet über Natriumsulfat. Nach dem Abdestillieren des Chloroforms löst man den Rückstand in Diäthyläther, fällt das Hydrazinderivat durch Einleiten von Chlorwasserstoff. Man saugt ab, behandelt den Rückstand mit Wasser, schüttelt mit Chloroform aus trocknet die Chloroform-Lösung und destilliert das Lösungsmittel ab.

Man erhält 4,5 g (73% d.Th.) eines orangefarbenen Öls.

Analyse:

| | | | |
|---|---|---|---|
| ber.: | C 54,7 | H 4,55 | Cl 13,65 |
| gef.: | C 54,11 | H 4,86 | Cl 13,36 |

*Beispiel 16*

2-Chlor-3-phenoxy-6-nitro-N-(6-aminohexan)-anilin

Eine Mischung aus 17,5 g 2-Chlor-4-nitro-1,3-diphenoxybenzol, 30 ml Dioxan und 9 g 1,6-Diaminohexan wird 4 Stunden bei 50°C gerührt, das Lösungsmittel abdestilliert und der Rückstand in Chloroform aufgenommenb. Man wäscht diese Lösung mit verdünnter Natronlauge und verdünnter Salzsäure, trocknet über Natriumsulfat, destilliert das Chloroform ab und nimmt den Rückstand in Diäthyläther auf, fällt mit Chlorwasserstoff, saugt ab und behandelt das abgetrennte halbfeste Produkt mit Chloroform/Sodalösung. Die Chloroformlösung wird abgetrennt, getrocknet und das Chloroform abdestilliert.

Ausbeute 12,5 g (69% d.Th.) eines viskosen gelben Öls.

Analyse:

| | | | |
|---|---|---|---|
| ber.: | C 59,3 | H 5,52 | Cl 11,55 |
| gef.: | C 59,1 | H 5,3 | Cl 11,72 |

Die in den nachstehenden Tabellen aufgeführten Verbindungen wurden nach folgenden allgemeinen Arbeitsweisen hergestellt:

Ausgangsstoffe:

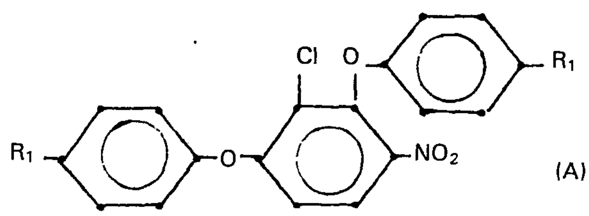

(A)

$R_1$: Chlor oder Wasserstoff

a) Umsetzung mit flüchtigen Aminen

(A) wird mit 10% Überschuss Amin in Dioxan 2 - 3 Tage stehen gelassen. Anschliessend wird eingedampft, der Rückstand in Äther aufgenommen und das entstandene Phenol durch Ausschütteln mit 2 n Natronlauge entfernt. Die ätherische Lösung wird mit Wasser zweimal geschüttelt, dann mit Natriumsulfat getrocknet und eingedampft.

b) Umsetzung mit nichtflüchtigen Aminen

(A) wird mit 100% Überschuss Amin in Dioxan 2 - 5 Stunden auf 80°C erwärmt. Nach dem Eindampfen wird der Rückstand in Äther aufgenommen und mit 2 n Natronlauge ausgeschüttelt. Eventuell vorhandenes Amin wird durch Ausschütteln mit 2 n Salzsäure entfernt. Nach dem Waschen mit Wasser wird die organische Phase mit Natriumsulfat getrocknet und eingedampft.

Tabelle I

Verbindungen der Formel

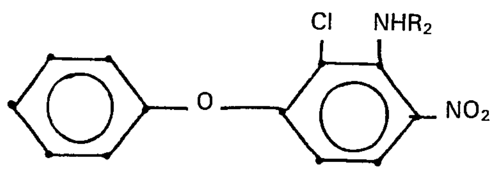

| Nr. | $R_2$ | Bedingungen | Ausbeute | RF-Werte 27°C Aceton/ Heptan 1 : 1 | Toluol | Fp |
|---|---|---|---|---|---|---|
| 1 | $CH_3$- | 2 Tage RT* | 100% ÖL | | | 72-74°C (aus Isopropylalkohol) |
| 2 | $C_2H_5$- | 3 Tage RT* | 83,5% Öl | 0,51 | 0,46 | |
| 3 | n-$C_3H_7$- | 2 Stdn. 80°C | 97,5% Öl | 0,615 | 0,47 | |
| 4 | i-$C_3H_7$- | 6 Stdn. 80°C | 94% Öl | 0,61 | 0,45 | |
| 5 | n-$C_4H_9$- | 2 Stdn. 80°C | 92% Öl | 0,625 | 0,50 | |
| 6 | i-$C_4H_9$ | 5 Stdn. 80°C | 96,5% Öl | 0,60 | 0,51 | |
| 7 | HO-$CH_2$-$CH_2$- | 2 Stdn. 80°C | 94% Öl | 0,445 | 0,01 | |
| 8 | -$CH(C_2H_5)_2$ | 5 Stdn. 80°C | 93% Öl | 0,47** | | |
| 9 | -$CH_2$-C≡CH | 5 Stdn. 80°C | 91% Öl | 0,31 | | |

* = Raumtemperatur
** = Toluol/Cyclohexan 1 : 1

Tabelle II

Verbindungen der Formel

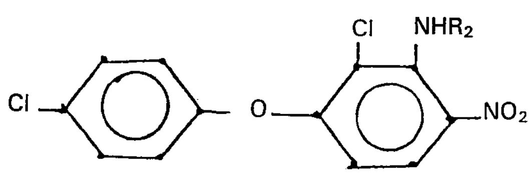

| Nr. | $R_2$ | Bedingungen | Ausbeute | RF-Werte 27°C Aceton/ Heptan 1 : 1 | Toluol | Fp |
|---|---|---|---|---|---|---|
| 1 | $CH_3$- | 2 Tage RT | 64% | | | 71-72°C (aus Isopropylalkohol) |
| 2 | $CH_2$=CH-$CH_2$- | 2 Tage RT | 98,5% | 0,52 | 0,51 | |
| 3 | -$CH(C_2H_5)_2$ | 3 Tage RT | 74% | | | |

Aus 2,4-Dichlor-6-nitro-1,3-diphenoxybenzol werden die in der Tabelle III aufgeführten Verbindungen der allgemeinen Formel

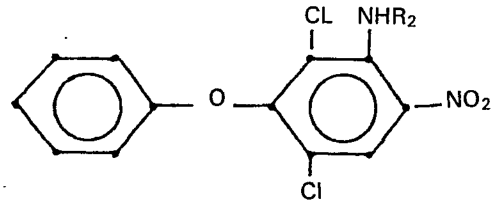

erhalten:

Tabelle III

| Nr. | $R_2$ | Amin/Reaktionsbedingungen | Ausbeute | Eigenschaften/Fp. |
|---|---|---|---|---|
| 1 | $CH_3$ | $NH_2CH_3$/24 Stdn. bei RT in Dioxan | 74% d.Th. | rote Kristalle Fp. 101°C |
| 2 | $C_2H_5$ | $NH_2C_2H_5$/16 Stdn. in Dimethylsulfoxyd | 85% d.Th. | hellbraunes Öl, dünnschicht-chromatograph. einheitlich |
| 3 | i-$C_3H_7$ | $NH_2$-i-$C_3H_7$/16 Stdn. bei RT in Dioxan | 88% d.Th. | orange Kristalle Fp. 83°C |
| 4 | n-$C_4H_9$ | $NH_2$-n-$C_4H_9$/2 Tage bei RT in Dioxan | 92% d.Th. | oranges Öl dünnschicht-chromatograph. einheitlich |
| 5 | n-$C_{12}H_{25}$ | $NH_2$-n-$C_{12}H_{25}$/5 Tage bei RT in Dioxan, Reinigung chromatographisch | 58% d.Th. | orange Kristalle Fp. 43°C |
| 6 | n-$C_{18}H_{37}$ | $NH_2$-n-$C_{18}H_{37}$/5 Tage bei RT in Dioxan, Reinigung chromatographisch | 51% d.Th. | 47 - 48°C |
| 7 | $-CH_2-CH_2-OH$ | $NH_2CH_2CH_2OH$/4 Tage bei RT in Dioxan | 100% d.Th. | dunkeles Öl |
| 8 | $-CH(C_2H_5)_2$ | $NH_2CH(C_2H_5)_2$/3 Tage bei RT in Dioxan | 63% d.Th. | |

Tabelle IV

Aus 1,3-Diphenoxybenzolen der Formel

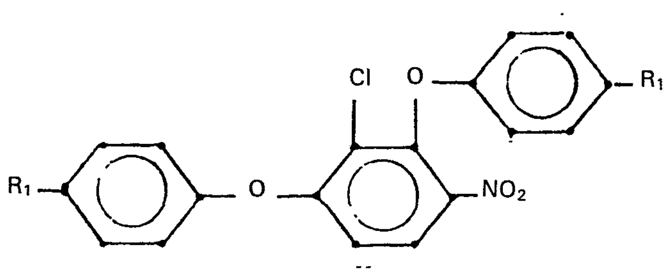

in denen $R_1$ die unten angegebene Bedeutung hat werden die nachstehenden Verbindungen der Formel

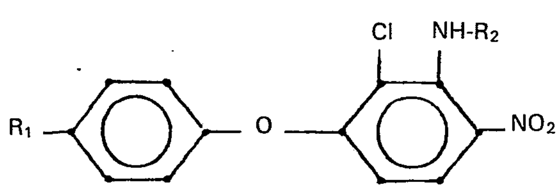

hergestellt:

| Nr. | $R_1$ | $R_2$ | Bedingungen | Ausbeute (% d.Th.) | RF-Werte 27°C Aceton/ Heptan 1:1 | Toluol | Fp |
|---|---|---|---|---|---|---|---|
| 1 | $CH_3$ | $CH_3$ | 2 Tage RT | 96% | 0,61 | 0,45 | 69-70°C |
| 2 | $CH_3$ | $CH_2=CH-CH_2-$ | 8 Stunden 60°C | 94% | 0,63 | 0,515 | (aus Isopropyl-Alkohol) |
| 3 | F | $C_2H_5$ | 3 Tage RT | 93% | | | |
| 4 | $OCH_3$ | $CH_3$ | 2 Tage RT | 89% | | | 100-101°C |
| 5 | $OCH_3$ | $n-C_3H_7$ | 3 Stunden 80°C | 95% | 0,59 | 0,29 | |

**Patentansprüche**

1. Verfahren zur Herstellung von Verbindungen der allgemeinen Formel

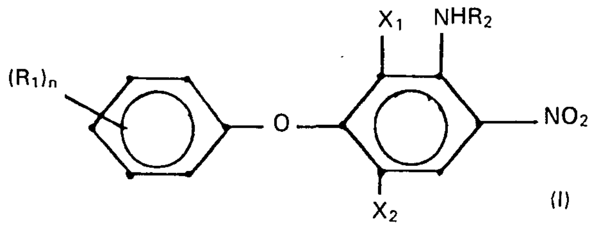

(I)

in der

n eine ganze Zahl von 1 bis 3,

$R_1$ Wasserstoff, Halogen, Alkyl mit 1 bis 8 C-Atomen, Trifluormethyl, Acetyl, CN, $C_1$-$C_4$-Alkoxy, $C_1$-$C_4$-Alkylthio,

$R_2$ Wasserstoff; Alkyl, Alkenyl oder Alkinyl mit bis zu 18 C-Atomen; einen durch Hydroxy, $C_1$-$C_4$-Alkoxy, Phenoxy, Halogenphenoxy, $C_1$-$C_4$-Alkylphenoxy, $C_1$-$C_4$-Alkoxyphenoxy, Nitrophenoxy, Cyanophenoxy, Amino oder $C_1$-$C_4$-Alkylthio substituierten Alkylrest mit 2 bis 6 C-Atomen; gegebenenfalls halogen- oder $CF_3$-substituiertes Benzyl, $NR_3R_4$, $CHR_3$-$COOR_4$ oder $CHR_3$-$CONR_4R_5$ bedeutet, wobei $R_3$, $R_4$ und $R_5$, die gleich oder verschieden sein können, Wasserstoff oder $C_1$-$C_8$-Alkylreste darstellen, und

$X_1$ und $X_2$, die gleich oder verschieden sein können, für Wasserstoff oder Halogen stehen,

dadurch gekennzeichnet, dass man ein Nitrodiphenoxybenzol der allgemeinen Formel

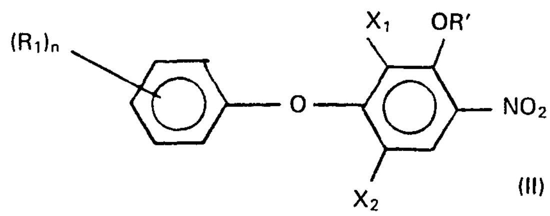

(II)

in der n, $R_1$, $R_2$, $X_1$ und $X_2$ die obige Bedeutung haben und R' für einen gegebenenfalls substituierten Phenylrest steht, mit einem Amin der allgemeinen Formel

$$HNHR_2 \qquad (III)$$

worin $R_2$ die obige Bedeutung hat, bei Temperaturen zwischen 20°C und 160°C umsetzt.

2. Verfahren nach Anspruch 1, dadurch gekennzeichnet, dass man als Ausgangsstoff eine Verbindung der allgemeinen Formel II einsetzt, in der R' die Gruppe

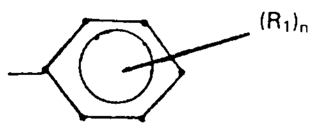

(IV)

bedeutet, worin $R_1$ und n die obige Bedeutung haben.

3. Verfahren nach Anspruch 1 oder 2, dadurch gekennzeichnet, dass man als Reaktionsmedium ein inertes organisches Lösungsmittel oder Wasser verwendet.

**Claims**

1. Process for preparing compounds of general formula

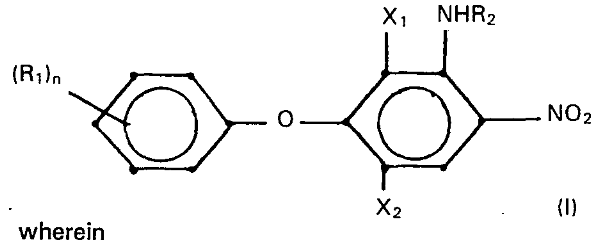

(I)

wherein

n is an integer from 1 to 3,

$R_1$ represents hydrogen, halogen, alkyl with 1 to 8 carbon atoms, trifluoromethyl, acetyl, CN, $C_{1\text{-}4}$ alkoxy or $C_{1\text{-}4}$ alkylthio,

$R_2$ represents hydrogen; alkyl, alkenyl or alkynyl with up to 18 carbon atoms; an alkyl group with 2 to 6 carbon atoms substituted by hydroxy, $C_{1\text{-}4}$ alkoxy, phenoxy, halophenoxy, $C_{1\text{-}4}$ alkylphenoxy, $C_{1\text{-}4}$ alkoxyphenoxy, nitrophenoxy, cyanophenoxy, amino or $C_{1\text{-}4}$ alkylthio; optionally halogen- or $CF_3$-substituted benzyl, $NR_3R_4$, $CHR_3$-$COOR_4$ or $CHR_3$-$CONR_4R_5$ wherein $R_3$, $R_4$ and $R_5$, which may be the same or different, represent hydrogen or $C_{1\text{-}8}$ alkyl groups, and

$X_1$ and $X_2$, which may be the same or different, represent hydrogen or halogen,

characterised in that a nitrodiphenoxybenzene of general formula

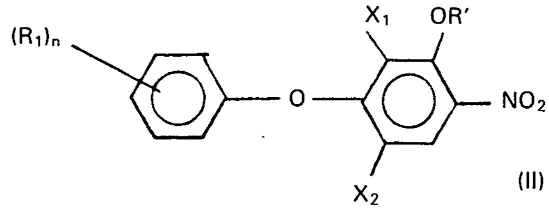

(II)

wherein n, $R_1$, $R_2$, $X_1$ and $X_2$ are as hereinbefore defined and R′ represents an optionally substituted phenyl group, is reacted with an amine of general formula

$HNHR_2$ (III)

wherein $R_2$ is as hereinbefore defined, at temperatures of between 20°C and 160°C.

2. Process as claimed in claim 1, characterised in that a compound of general formula II wherein R′ represents the group

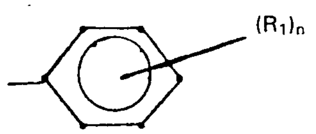

(IV)

(wherein $R_1$ and n are as hereinbefore defined) is used as starting material.

3. Process as claimed in claim 1 or 2, characterised in that an inert organic solvent or water is used as the reaction medium.

**Revendications**

1. Procédé pour la préparation de composés de formule générale

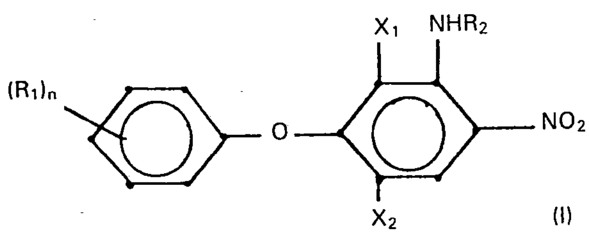

(I)

dans laquelle

n représente un nombre entier de 1 à 3,

$R_1$ représente hydrogène, halogène, alcoyle avec 1 à 8 atomes de C, trifluorméthyle, acétyle, CN, alcoxy en $C_1$-$C_4$, alcoylthio en $C_1$-$C_4$,

$R_2$ représente hydrogène; alcoyle, Alcenyle ou alcynyle avec jusqu'à 18 atome de C; un radical alcoyle avec 2 à 6 atomes de C substitué par hydroxy, alcoxy en $C_1$-$C_4$, phénoxy, halogénophénoxy, alcoyle en $C_1$-$C_4$-phénoxy, alcoxy en $C_1$-$C_4$-phénoxy, nitrophénoxy, cyanophénoxy, amino ou alcoylthio en $C_1$-$C_4$; benzyle éventuellement halogénosubstitué ou substitué par $CF_3$, $NR_3R_4$, $CCHR_3$-$COOR_4$ ou $CHR_3$-$CONR_4R_5$, $R_3$, $R_4$ et $R_5$ qui peuvent être identiques ou différents, représentant l'hydrogène ou des radicaux alcoyle en $C_1$-$C_8$, et

$X_1$ et $X_2$ qui peuvent être identiques ou différents, remplacent hydrogène ou halogène,

caractérisé en ce que l'on fait réagir un nitrodiphénoxybenzène de formule générale

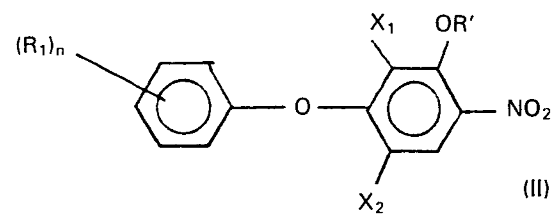

(II)

dans laquelle n, $R_1$, $R_2$, $X_1$ et $X_2$ ont la signification ci-dessus et R′ représente un radical phényle éventuellement substitué, avec une amine de formule générale

$HNHR_2$ (III)

dans laquelle $R_2$ a la signification ci-dessus, à des températures entre 20°C et 160°C.

2. Procédé selon la revendication 1, caractérisé en ce que l'on met en œuvre en tant que matière de départ un composé de formule générale II dans laquelle R′ représente le groupe

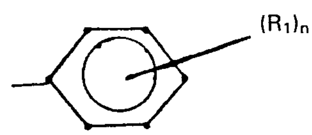

(IV)

dans lequel $R_1$ et n ont la signification ci-dessus.

3. Procédé selon la revendication 1 ou 2, caractérisé en ce que l'on utilise comme milieu réactionnel un solvant organique inerte ou de l'eau.